# FASCICULE DE BREVET EUROPEEN

(11) **EP 3 169 156 B1**
(45) Date de publication et mention de la délivrance du brevet: **25.07.2018**
(21) Numéro de dépôt: 15738721.8
(22) Date de dépôt: 17.06.2015
(51) Int. Cl.: A61K 31/7048, A61K 31/7052, A61K 31/215, A61K 31/496, A61K 31/65, A61K 45/06, A01N 37/44, A01N 43/90, A61P 31/00, A61P 33/14

(54) **ASSOCIATION D'IVERMECTINE ET DE COMPOSÉ(S) ANTIBIOTIQUE(S) POUR UN TRAITEMENT THÉRAPEUTIQUE CONTRE LES POUX**
ASSOZIATION VON IVERMECTIN UND ANTIBIOTISCHEN VERBINDUNGEN ZUR THERAPEUTISCHEN BEHANDLUNG VON LÄUSEN
ASSOCIATION OF IVERMECTIN AND ANTIBIOTIC COMPOUNDS FOR THE THERAPEUTIC TREATMENT OF LICE

(30) Priorité: 18.07.2014 FR 1456986
(43) Date de publication de la demande: 24.05.2017
(73) Titulaire: Fondation Méditerranée Infection, 13005 Marseille (FR); Université d'Aix-Marseille, 13284 Marseille Cedex 07 (FR)
(72) Inventeur: RAOULT, Didier, F-13008 Marseille (FR); ROLAIN, Jean-Marc, F-13006 Marseille (FR); SANGARE, Abdoul Karim, Bamako (ML)
(74) Mandataire: Domange, Maxime
(86) Numéro de dépôt international: PCT/FR2015/051604
(87) Numéro de publication internationale: WO 2016/009119

(56) Documents cités:
- EP-A1- 0 238 259
- WO-A1-03/024437
- WO-A1-2005/007188
- KHAN ET AL: "Effect of some antibiotics on a microorganism isolated from the mycetome of Pediculus humanus capitis", FERTILIZER TECHNOLOGY, FERTILIZER CO. OF INDIA, SINDRI, IN, vol. 14, no. 3, 1 janvier 1977 (1977-01-01), pages 274-275, XP008175362, ISSN: 0378-0430
- DAVID M. PARISER ET AL: "Topical 0.5% Ivermectin Lotion for Treatment of Head Lice", NEW ENGLAND JOURNAL OF MEDICINE, vol. 367, no. 18, 1 novembre 2012 (2012-11-01), pages 1687-1693, XP055079450, ISSN: 0028-4793, DOI: 10.1056/NEJMoa1200107
- Cédric Foucault ET AL: "Oral Ivermectin in the Treatment of Body Lice", JID, 1 février 2006 (2006-02-01), pages 474-476, XP055207613, Extrait de l'Internet: URL:http://jid.oxfordjournals.org/content/ 193/3/474.full.pdf [extrait le 2015-08-13]
- Marcia L. Buck: "Doxycycline for Pediatric Infections", Pediatr. Pharm. Pediatr. Pharm., vol. 9, no. 10 2003, 2003, pages 1-5, XP002743345, Extrait de l'Internet: URL:http://www.medscape.com/viewarticle/46 3541 [extrait le 2015-08-13]
- DAVIDE SASSERA ET AL: "Microbial symbiosis and the control of vector-borne pathogens in tsetse flies, human lice, and triatomine bugs", PATHOGENS AND GLOBAL HEALTH, vol. 107, no. 6, 1 septembre 2013 (2013-09-01), pages 285-292, XP055174572, ISSN: 2047-7724, DOI: 10.1179/2047773213Y.0000000109
- Anonymous: "Clinically obsolete antibiotics could be used to treat head and body lice", , 23 juin 2010 (2010-06-23), pages 1-2, XP055176244, Extrait de l'Internet: URL:http://phys.org/news196521251.html [extrait le 2015-03-13]
- GIANNELLI ALESSIO ET AL: "Treatment ofDirofilaria repensmicrofilariaemia with a combination of doxycycline hyclate and ivermectin", VETERINARY PARASITOLOGY, vol. 197, no. 3, 2013, pages 702-704, XP028733087, ISSN: 0304-4017, DOI: 10.1016/J.VETPAR.2013.05.012
- AHMED EL-TAHTAWY ET AL: "The Effect of Azithromycin on Ivermectin Pharmacokinetics-A Population Pharmacokinetic Model Analysis", PLOS NEGLECTED TROPICAL DISEASES, vol. 2, no. 5, 14 mai 2008 (2008-05-14), pages 1-10, XP055207647, DOI: 10.1371/journal.pntd.0000236

## Description

La présente invention concerne l'utilisation de composés antibiotiques tels que définis dans les revendications en association avec un composé antiparasitaire consistant dans l'ivermectine, pour une application thérapeutique nécessitant une action létale contre les poux. L'invention concerne également une association pour une application nécessitant une action létale contre les poux comprenant au moins un composé antibiotique choisi parmi la doxocycline, la rifampicine, l'érythromycine et l'azithromycine, et un composé antiparasitaire consistant dans l'ivermectine, tel que défini dans les revendications. Les poux de corps sont un problème majeur de santé publique. Il existe 3 types de poux : le pou de tête (*Pediculus humanus capitus*), le pou de corps (*Pediculus humanus humanus*) qui appartiennent à la famille des *Pediculidae* et le pou pubien (*Phtirius pubis*) qui est de la famille des *Phtiridae.*

On estime que jusqu'à 30% des élèves des écoles, en France, sont porteurs de poux. Les poux posent un problème majeur, actuellement, du fait de leur résistance aux insecticides. Ainsi, le traitement le plus communément utilisé, la perméthrine, a provoqué des résistances chez les poux qui dans certaines populations peuvent atteindre 100% des poux exposés à la perméthrine.

Dans ces conditions, il est nécessaire de trouver des traitements alternatifs. Parmi les traitements alternatifs, il a été proposé d'utiliser l'ivermectine comme traitement per os (1,2) ou sous forme d'onguent. Les poux étant des hématophages stricts, en prenant le repas sanguin avec de l'ivermectine, il se produit une action directe inhibitrice entre les fibres nerveuses et les cellules musculaires du pou par l'intermédiaire du neuromédiateur GABA ce qui est mortel pour les poux par paralysie. Ce traitement permet aussi de tuer les larves qui résident dans les oeufs des poux car les larves sont aussi des hématophages stricts dès leur naissance alors que les oeufs ne sont pas susceptibles d'être atteints par l'ivermectine. Ce traitement est relativement efficace en administration *per os.* Cependant, il subsiste le problème de la résistance à l'ivermectine. En effet, dans les traitements utilisant l'ivermectine les poux se sont montrés rapidement résistants (3). Le document WO-A-2005/007188 divulgue l'utilisation d'un chélateur métallique et/ou un inhibiteur de métalloprotéase pour le traitement d'infestations ectoparasitaires, comme par exemple les poux. Le but de la présente invention est de trouver un nouveau traitement des maladies liés aux poux.

Selon la présente invention, on a découvert qu'un traitement antibiotique pouvait être efficace sur les poux, et permettait de les tuer, en tirant parti de ce que les poux ont une bactérie qui vit en symbiose avec eux, qui s'appelle *Candidatus Riesia pediculosa.* Le pou de corps comprend des organes appelés mycétomes qui abritent cette bactérie endosymbionte. Cette bactérie est nécessaire aux poux, en lui apportant de la vitamine B (4, 11).On a démontré selon la présente invention qu'un antibiotique a un effet direct sur le mycétome des poux et on a démontré qu'en tuant ladite bactérie qu'ils contiennent, il en résulte une action létale des antibiotiques sur les poux et une diminution de leur reproduction.

Cette invention a nécessité de mettre au point une technique pour nourrir les poux sur une membrane artificielle avec du sang humain en lui faisant prendre son repas à travers un film de paraffine. Ce système a permis de nourrir les poux, de manière quotidienne, sur du sang humain. Cette préparation permet d'incorporer des quantités de différents composés tels que antibiotiques et/ou d'ivermectine aux repas sanguins des poux, et de mesurer leur survie. Par ailleurs, selon la présente divulgation on a développé une méthode de microscopie confocale et analyse par déconvolution spectrale décrite ci-après permettant de mesurer la survie de la bactérie *Candidatus Riesia pediculosa* en mesurant la quantité d'ARN qu'elle sécrète, par une technique d'hybridation in situ détectant les copies d'ARN par fluorescence (FISH = Fluorescent In Situ Hybridization). Les copies d'ARN témoignent de la viabilité de la bactérie.

La présente invention a pour objet l'association (a) d'au moins un composé antibiotique choisi parmi la doxycycline, la rifampicine, l'érythromycine et l'azithromycine et (b) d'un composé antiparasitaire consistant dans l'ivermectine pour une utilisation dans un traitement thérapeutique contre les poux notamment pour le traitement d'un patient humain ou d'un animal notamment un mammifère.

Plus particulièrement, l'application thérapeutique visée est le traitement des pédiculoses corporelle ou capillaire liées à la présence de poux.

Plus particulièrement l'application est une action létale contre les poux et/ou une action contre leur reproduction c'est-à-dire diminuant leur capacité de reproduction.

Lesdits composés antibiotiques selon l'invention sont des composés antibiotiques bactéricides à activité intracellulaire, notamment choisi parmi les familles des tétracyclines pour la doxycycline, des macrolides pour l'érythromycine et l'azithromycine et des rifamycines pour la rifampicine.

On a obtenu un effet de diminution significative de l'espérance de vie des poux et de leur capacité à pondre des oeufs à partir de concentrations d'antibiotiques dans le sang humain d'au moins 4 µg/ml, de préférence au moins 10 µg/ml, la différence étant plus significative à partir de 20 µg/ml voire au moins 50 µg/ml et ce aussi bien sur la diminution de la ponte que sur la diminution de la durée de survie des poux.

Plus particulièrement, la présente invention a pour objet, l' association pour une application nécessitant une action létale contre les poux comprenant au moins un dit composé antibiotique et un dit composé antiparasitaire à activité létale contre les poux pour une utilisation simultanée, séparée ou étalée dans le temps des dits(s) composé(s) antibiotique(s) et dudit composé anti parasitaire.

Selon la présente invention on a mis en évidence un effet synergique de cette association à savoir un effet supérieur par rapport à ceux du dit composé antibiotique pris seul et du dit composé anti parasitaire pris seul. En outre, l'intérêt d'associer puiseurs composés est de diminuer le risque d'apparition de mutants résistants.

Ledit composé antiparasitaire est un composé de la famille des composés antihelminthiques de type avermectine. Lesdits composés antihelminthiques sont administrables par voie interne chez l'homme.

L'ajout de doxycycline, rifampicine, érythromycine ou azithromycine donne des résultats qui sont comparables, avec au moins au départ, une activité supérieure lorsqu'on associe l'ivermectine avec la rifampicine ou la doxycycline. Toutefois, la doxycycline est contre-indiquée chez l'enfant car elle empêche une bonne formation de l'émail dentaire et colore les dents en jaune. Les autres antibiotiques peuvent être utilisés aussi bien chez l'adulte que chez l'enfant.

En conséquence, de préférence, dans une association pour une utilisation pour le traitement d'un enfant de moins de 15 ans, ledit composé antibiotique est autre que la doxycycline.

De préférence, pour le traitement d'un enfant de moins 15 ans, le composé antibiotique est choisi parmi l'érythromycine et l'azithromycine. En effet, la rifampicine peut provoquer des effets secondaires indésirables tels que coloration des muqueuses notamment avec coloration des urines et des yeux de couleur orange.

La probabilité de sélectionner des mutants résistants est le produit de la probabilité de sélection de chacun des composés : si 1 chance sur 10⁻ⁿ pour l'un et une chance pour 10^{-p} pour l'autre alors la probabilité d'avoir dans une population donnée un pou résistant aux deux est de 10^{-(n+p)}.

On a pu mettre en évidence que les antibiotiques doxycycline, rifampicine, azithromycine et érythromycine ont une activité létale pour le pou et une action synergique en association avec l'ivermectine, à des concentrations d'antibiotiques dans le sang humain d'au moins 4 µg/ml, de préférence au moins 10 µg/ml, notamment de 4 à 50 µg/ml, L'ivermectine donnant des résultats à partir de 50 ng/ml dans le sang humain et de préférence à la concentration de 50 ng/ml à 200 ng/ml.

Ces concentrations sanguines sont susceptibles d'être atteintes par la prescription de comprimés administrables *per os* (oralement) à des doses journalières comme suit :
- une dose d'ivermectine de 100 à 300 µg/kg/jour (rapportée au poids du patient), de préférence inférieure à 200 µg/kg/jour (posologie usuelle),
- une dose de 125 à 300 mg/jour de doxycycline, de préférence inférieure à 200 mg/jour (posologie usuelle),
- une dose de 5 à 30 mg/kg/jour (rapportée au poids du patient adulte ou enfant de moins de 15 ans) de rifampicine, de préférence inférieure à 20 mg/kg/jour,
- une dose d'érythromycine de 1 à 3 g/jour chez l'adulte, de préférence inférieure à 2g/jour,
- une dose d'érythromycine de 20 à 50 mg/kg/jour chez l'enfant de moins de 15 ans, de préférence inférieure à 30 mg/kg/jour,
- une dose d'azithromycine de 250 à 750mg/jour chez l'adulte, de préférence inférieure à 500mg/jour, et
- une dose d'azithromycine de 10 à 25 mg/kg/jour chez l'enfant de moins de 15 ans, de préférence inférieure à 20 mg/kg/jour chez l'enfant de moins de 25 kg sans dépasser la posologie de 500 mg/jour.

Les concentrations efficaces d'antibiotiques et d'ivermectine données ci-dessus sont les concentrations pertinentes optimales dans le cadre d'une application en association synergique entre antibiotique et ivermectine et/ou également lorsque lesdits composés sont pris seuls sans association étant entendu que la concentration efficace de l'antibiotique et du composé anti parasitaire sont inférieures lorsque lesdits composés sont pris en association.

Dans un mode de réalisation, on met en oeuvre une association comportant une pluralité de composés antibiotiques différents avec un seul composé antiparasitaire.

La présente divulgation a donc également pour objet une composition pharmaceutique sous forme administrable par voie orale ou par application topique cutanée ou capillaire comprenant un dit composé antibiotique en quantité thérapeutiquement efficace pour application thérapeutique antiparasitaire contre les poux selon l'invention.

Plus particulièrement, la composition pharmaceutique sous forme administrable par voie orale ou par application topique cutanée ou capillaire comprend un composé antibiotique combiné à un composé antiparasitaire en quantité thérapeutiquement efficace pour une association pour application thérapeutique antiparasitaire contre les poux selon l'invention.

Plus particulièrement encore, la composition pharmaceutique comprend un dit composé antibiotique qui est choisi parmi les doxycycline, rifampicine, érythromycine et azithromycine, de préférence choisi parmi la doxycycline et la rifampicine pour lesquels la synergie est la plus forte pour traiter un adulte et choisi parmi l'érythromycine et l'azithromycine pour traiter un enfant.

Plus particulièrement encore, la composition pharmaceutique se présente dans une forme galénique choisie parmi les formes orales liquides ou solide, de préférence sous forme de comprimé ou gélule, et les formes cutanées, de préférence sous forme de pommade, pate, crème, lotion, aérosol, savon et shampoing.

La présente invention a donc également pour objet un ensemble de compositions pharmaceutiques pour une utilisation selon l'invention, notamment sous forme de trousse ou kit, caractérisé en ce qu'il comprend une pluralité de doses journalières ou doses unitaires de compostions pharmaceutiques des dit(s) composé(s) antibiotique(s) et dit composé antiparasitaire en quantités utiles pour être appliquées dans un traitement de plusieurs jours, comprenant :
- une pluralité de doses unitaires ou journalières de compositions pharmaceutiques comprenant un dit composé anti parasitaire, et
- une pluralité de doses unitaires ou journalières de compositions pharmaceutiques comprenant au moins un dit composé antibiotique.

Plus particulièrement, l'ensemble de compositions pharmaceutiques comprend une pluralité de doses unitaires de dits composé antibiotique et composé antiparasitaire en quantités utiles pour être appliquées dans un traitement de plusieurs jours, comprenant :
- une pluralité de doses unitaires ou journalières de compositions pharmaceutiques d'un mélange de dits composé(s) antibiotique(s) et de dit composé antiparasitaire, et
- une pluralité de doses unitaires ou journalières de compositions pharmaceutiques de composé(s) antibiotique(s) comme seul agent actif létal contre les poux.

Plus particulièrement encore, l'ensemble de compositions pharmaceutiques comprend un dit composé antibiotique et un dit composé antiparasitaire en quantités utiles pour être appliquées dans un traitement de 7 jours, comprenant :
- deux doses unitaires journalières de mélange d'ivermectine et d'au moins un composé antibiotique choisi parmi la doxycycline, la rifampicine, l'érythromycine et l'azithromycine, pour les 1er et 7ème jours,
- 5 doses unitaires journalières d'au moins un composé antibiotique choisi parmi la doxycycline, la rifampicine, l'érythromycine et l'azithromycine, sans ivermectine, pour respectivement les 5 jours suivants le premier jour.

Deux doses d'ivermectine à 7 jours d'intervalle associé à un antibiotique tous les jours pendant 7 jours permettent de s'assurer d'avoir tué tous les poux mais aussi les larves. Il n'a pas été démontré d'intérêt d'une deuxième dose d'ivermectine avant une semaine pour tuer les larves qui éclosent. En revanche, les traitements à base d'antibiotique sont généralement de 5 à 7 jours consécutifs.

En particulier, les inventeurs ont réalisés avec succès des traitements de 7 jours, comprenant :
- une combinaison d'ivermectine et un seul dit composé antibiotique le 1er jour,
- un seul composé antibiotique sans ivermectine pendant les 5 jours suivants, et
- un 2ème traitement avec une combinaison d'ivermectine et un seul dit composé antibiotique le 7ème jour.

Ceci permet d'une part, d'être plus efficace que les traitements existants actuellement du fait d'un effet synergique, et d'autre part d'empêcher ou de réduire l'apparition de mutants résistant à l'ivermectine et/ou aux antibiotiques.

D'autres caractéristiques et avantages de la présente invention ressortiront mieux à la lecture des exemples de la description qui va suivre, faite de manière illustrative, en référence aux dessins annexés sur lesquels :

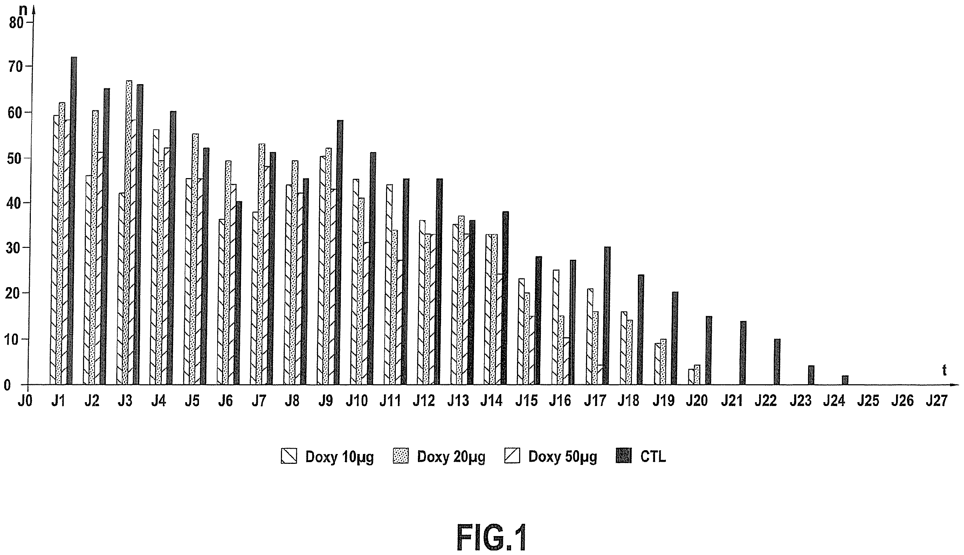

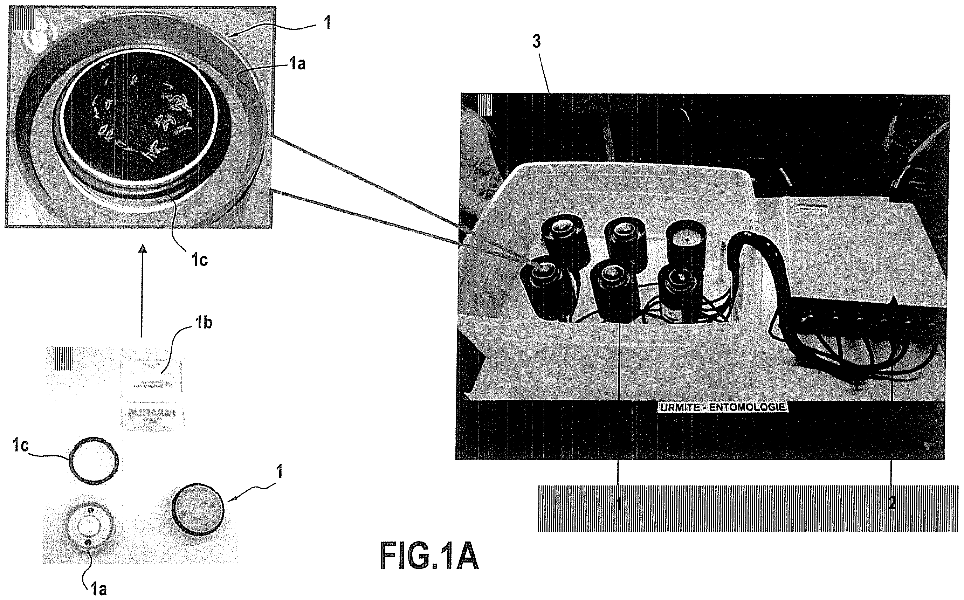

- la figure 1A représente une installation d'alimentation des poux en sang ;
- la figure 1 représente les nombres de lentes récupérées par jour dans les 4 groupes traités à l'exemple 1 avec du sang contenant de la doxycycline à 10 µg/ml, 20 µg/ml et 50 µg/ml et respectivement un groupe contrôle sans doxycycline ; n= nombre de lentes récupérées du jour J0 à J24 ;

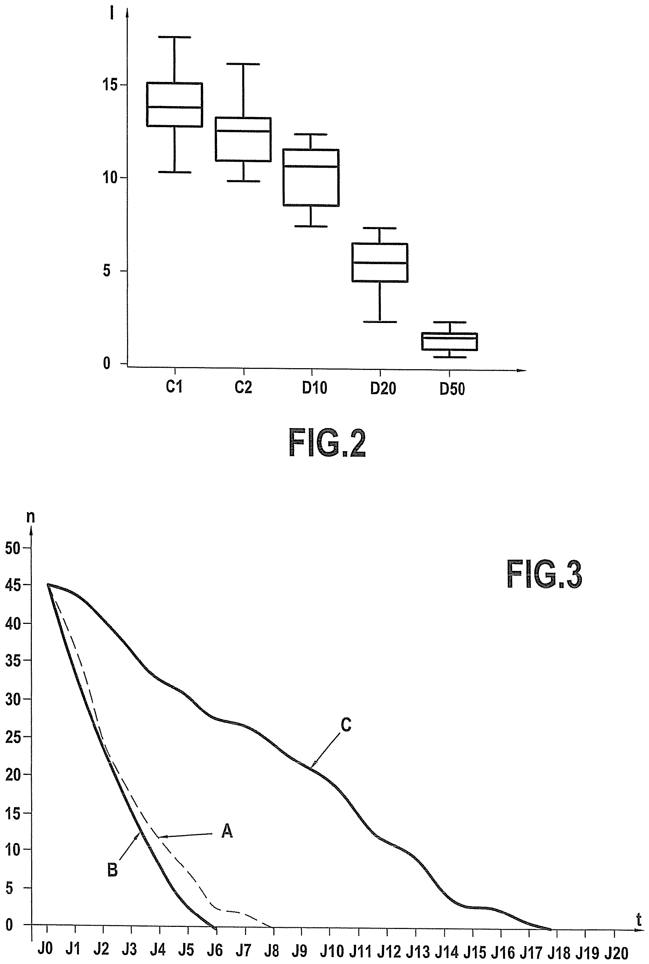

- la figure 2 représente les mesures d'intensités moyennes de fluorescence (I) de groupes de poux traités à l'exemple 1 (C1=contrôle de poux vivants, C2= contrôle de poux morts, D10= poux traités avec 10 µg/ml de doxycycline, D20= poux traités avec 20 µg/ml de doxycycline, D50= poux traités avec 50 µg/ml de doxycycline) ;
- la figure 3 représente les courbes de survie des poux traités à l'exemple 2 au cours du temps t (J0 à J18) avec l'ivermectine seule à 50 ng/ml(A) et l' association ivermectine 50ng/ml/ doxycycline 20ug/ml (B), et avec du sang sans antibiotique (C=contrôle), n= nombre de poux survivant aux jours J0 à J18.

### Exemple 1 : traitement des poux par alimentation avec du sang contenant la doxycycline (référence).

### 1) Matériels

### 1.1) Souche de pou de corps.

Une lignée pure de longue date de pou du corps humain, (P. h. Corporis, souche Orlando), élevés sur des lapins dans le laboratoire des inventeurs a été utilisé comme modèle dans cet exemple (5-7).

### 1.2) Installation d'alimentation des poux.

Pour l'alimentation en sang des poux, on a réalisé une installation 3 montrée figure 1A mettant en oeuvre un dispositif appelé Hemotek ™5W1 membrane feeding system basé sur une technologie d'alimentation à travers une membrane. L'installation comprend une unité de puissance 2 qui délivre du courant électrique (PS5A/220 Power Unit 5W1 System, Accrington Lancs, Angleterre), BB5 6JZ Angleterre) et cinq dispositifs d'alimentation 1 équipés de moyens de contrôle de température électronique et de moyens de chauffage. Le dispositif d'alimentation 1 comporte un réservoir de sang la, qui est amovible pour faciliter le nettoyage et le passage à l'autoclave si nécessaire. La température de fonctionnement de chaque dispositif d'alimentation peut être ajustée en fonction de la préférence d'espèces d'insectes. Un carré de membrane d'alimentation synthétique 1b (Parafilm (US), Chicago IL.60631) mesurant environ 3x3 cm est tendu au-dessus et autour de l'ouverture du réservoir de repas de sang et fixé par un anneau 1c. Le réservoir est rempli de sang à l'aide d'une pipette de transfert de façon à ce que la membrane affleure en contact avec la surface du sang. Ainsi la membrane joue le rôle de la peau et le sang la traverse par capillarité et la surface de la membrane est suffisante pour recevoir 100 poux 3.

### 2) Méthodes.

### 2.1) Préparation des repas de sang avec de la doxycycline.

Du sang humain Groupe A rhésus positif (A +) n° 53332 de l'Établissement Français du Sang (EFS) a été utilisé. Le Sang a été distribué dans les tubes de 5 ml et conservé à -20° C avant de l'utiliser. D'abord il est chauffé dans un bain humide à 37° C pendant 30 minutes. Puis, dans un volume de 1,485 ml de sang hémolysé, pour chacun des trois groupes de poux, il a été ajouté 15 µl de doxycycline (20 mg / ml) (Vibraveineuse ® Lot: A262408, Laboratoires SERB, Paris, France) à des concentrations différentes de 10, 20 et 50 µg / ml. Le volume final de 1,5 ml obtenu pour chaque concentration a été introduit dans les différents réservoirs.

### 2.2) Suivi des poux.

280 poux adultes (comprenant des mâles et des femelles) répartis en 4 groupes de 70 poux ont été mis sur un morceau de tissu noir conservé dans des pots et maintenu à l'étuve à 29° C et 90% d'humidité. Trois groupes de poux ont été nourris avec du sang contenant différentes concentrations de doxycycline (10, 20 et 50 µg/ml). Un quatrième groupe de poux représente un témoin négatif alimenté avec du sang sans doxycycline. Les poux ont été mis tous les jours sur la membrane pour une heure de repas de sang. Chaque jour, les poux morts sont récupérés de chaque lot et conservés dans la solution de fixation pour conserver les poux après en avoir enlevé les pattes pour faciliter l'infiltration de réactifs. Puis, la survie et la mortalité des poux et les lentes pondues ont été notées et comptées chaque jour, dans chacun des groupes. Cette expérience a été reproduite trois fois.

### 2.3) Hybridation et fluorescence in situ (FISH).

On a collecté dans chaque groupe un pou mort par jour pendant 10 jours. En plus, dix autres poux vivants ont été sacrifiés en tant que témoins vivants. Tous ces échantillons ont été conservés directement dans le produit de fixation Carnoy (chloroforme-éthanol-acide acétique, 06:03:01). Après fixation, les échantillons ont été décolorés afin de faciliter l'hybridation et obtenir une meilleure fluorescence dans 6% d'H2O2 dans l'éthanol pendant 2 heures, puis mis en hybridation avec un tampon (20 mM Tris-HCl [pH 8,0], 0,9 M de NaCl, 0,01% de dodécylsulfate de sodium, 30% de formamide) contenant 10 pmol de sondes fluorescentes/ml basées sur les séquences ARNr 16S de Candidatus Riesia pediculicola, Cy3 Lice1255R (5-CTTGGCTCGCTCTTACGAGT-3). Après la dénaturation des échantillons à 65° C pendant 15 minutes, ils ont été incubés pendant une nuit dans la solution d'hybridation. Au jour 2, les poux ont été retirés de la solution d'hybridation, puis lavé trois fois avec une solution de lavage (NaCl 0,3 M, citrate de sodium 0,03 M, 0,01% de dodécylsulfate de sodium) pendant 5 minutes. Ensuite, ils ont été laissés dans une solution de 4',6-diamidino-2-phénylindole (DAPI) deux à trois minutes. Ensuite, les lames sont conservées à l'abri de la lumière.

### 2.4) Microscopie confocale.

On a utilisé la technique de microscopie confocale avec analyse par déconvolution spectrale (Confocal Application Letter 23, Oct. 2008 N°30) dont le principe consiste à différencier plusieurs émissions de lumière excitées avec la même longueur d'onde. Lors de l'acquisition d'images avec deux fluorochromes possédant des spectres très proches, il est nécessaire de séparer les deux fluorochromes grâce au mode de détection spectrale du confocal (λ scan).Le principe est d'acquérir les deux spectres de chacun des fluorochromes situés sur des lames distinctes, puis d'acquérir le spectre d'une lame doublement marquée. Par traitement logiciel, les deux marquages seront séparés spectralement.

Afin de différencier l'auto-fluorescence due à la chitine des poux de la fluorescence propre de la sonde ADN, on a d'abord marqué et monté le pou entre lame et lamelle, et l'imagerie a été faite avec un microscope à résonance confocale de type Leica SP5 AOBS en mode lambda. Ensuite, l'échantillon de poux a été scanné en 32 acquisitions d'une largeur spectrale de 5nm sur une bande de 160nm (32 images x 5nm de largeur spectrale= 160nm) en prenant soin de ne jamais saturer le signal. La mesure de la fluorescence a été définie comme étant l'extraction de deux images distinctes, une pour la fluorescence et une pour l'auto-fluorescence. Les paramètres de réglages du microscope étant faits, toutes les acquisitions ont été réalisées en respectant les mêmes paramètres de réglage (gain, offset, puissance du laser et objectif). Pour chaque série d'acquisition d'images, on a utilisé le logiciel MetaMorph cversion 4.6, Molecular Devices, USA) pour calculer les ratios des intensités entre les deux signaux de fluorescence pour chaque pou.

### 2.5) Analyse statistique.

L'analyse a été effectuée avec le logiciel R version 2.15.2 (R Develpment Core Team, 2013). Les tests statistiques ont été effectués de manière bilatérale et les valeurs de p ≤ 0,05 ont été considérées comme significatives.

### B) Résultats.

Pour standardiser cette technique, on a utilisé trois lots de contrôles de quarante poux nourris à J0 avec du sang humain sans doxycycline. Chaque groupe de poux a été suivi jusqu'à terme (tableau S1 ci-après) et le pourcentage de poux gorgés était supérieur à 90% ce qui signifie que le système d'alimentation des poux fonctionne bien. Le nombre de poux diminue au cours du temps du fait du manque d'alimentation des poux et chaque pou a une durée de vie qui lui est propre, même si les éclosions ont été faites à la même date.

| **Tableau S1. surveillance des poux vivants récupérés par jour pendant la standardisation des 3 lots contrôles** | | | |
|---|---|---|---|
| **jours** | **Lot 1** | **Lot 2** | **Lot 3** |
| **J0** | 40 | 40 | 40 |
| **J1** | 38 | 33 | 36 |
| **J2** | 35 | 30 | 33 |
| **J3** | 32 | 26 | 27 |
| **J4** | 29 | 24 | 26 |
| **J5** | 25 | 19 | 25 |
| **J6** | 19 | 12 | 19 |
| **J7** | 16 | 9 | 12 |
| **J8** | 13 | 7 | 9 |
| **J9** | 9 | 3 | 6 |
| **J10** | 7 | 1 | 5 |
| **J11** | 5 | 0 | 3 |
| **J12** | 4 | | 3 |
| **J13** | 2 | | 0 |
| **J14** | 1 | | |
| **J15** | 0 | | |
| **J16** | | | |

Ce travail a donc permis de développer un nouveau modèle d'alimentation pour les poux et a également permis de démontrer la possibilité de les nourrir avec un antibiotique ici la doxycycline à travers une membrane artificielle.

La doxycycline réduit la durée de vie et la fertilité des poux ce qui démontre que les symbiontes sont impliqués dans les fonctions vitales de leurs hôtes. Pour déterminer si la doxycycline a un effet sur la durée de vie et la fertilité des poux, on a suivi trois groupes de poux sous traitement à la doxycycline à 3 doses différentes (10, 20 et 50 µg/ml dans le sang) et un contrôle négatif. Les résultats montrent que la survie des poux dans le groupe 3 avec 50 µg de doxycycline diminue plus rapidement par rapport aux groupes de 10 µg, 20 µg/ml et au contrôle négatif (Tableau 1 ci-après).

Une concentration élevée (50 µg/ml) de doxycycline agit rapidement sur la durée de vie des poux. Les médianes de survie (MS) et leurs intervalles de confiance (IC) sont respectivement: groupe témoin (MS = 13,0 jours; IC = [11-16] jours), groupe avec doxycycline 10 µg/ml (MS = 8,0 jours; IC = [7 - 11] jours), groupe avec 20 µg/ml (MS = 8,0 jours; CI = [6-11] jours), groupe avec doxycycline 50 µg/ml (MS = 6,5 jours; IC = [5-8] jours).

Cependant, on observe que l'effet toxique de la doxycycline ne diffère pas entre 10 et 20 µg/ml doses (Logrank test p = 0,90). On a également comparé la survie moyenne dans les différents groupes. Les résultats montrent que la survie moyenne diffère selon le groupe (p = 9.5e-07, test de Kruskal-Wallis), mais n'a toutefois pas différé entre les groupes doxycycline 10 et 20 µg/ml (p-value = 0,92, Student's t test avec approximation de Welch). Ainsi, ces résultats démontrent l'effet significatif de la doxycycline sur la mortalité des poux (p-value = 3.7x10-10, Test Logrank).

| **Tableau 1. surveillance des poux vivants récupérés par jour** | | | | |
|---|---|---|---|---|
| | **Doxy 10 µg/ml** | **Doxy 20 µg/ml** | **Doxy 50 µg/ml** | **C** |
| **J0** | 70 | 70 | 70 | 70 |
| **J1** | 69 | 68 | 69 | 70 |
| **J2** | 68 | 65 | 64 | 69 |
| **J3** | 61 | 55 | 58 | 66 |
| **J4** | 53 | 51 | 50 | 62 |
| **J5** | 48 | 48 | 43 | 59 |
| **J6** | 46 | 44 | 38 | 56 |
| **J7** | 43 | 40 | 35 | 54 |
| **J8** | 36 | 36 | 32 | 53 |
| **J9** | 29 | 32 | 25 | 51 |
| **J10** | 28 | 29 | 21 | 49 |
| **J11** | 27 | 27 | 17 | 46 |
| **J12** | 23 | 25 | 16 | 42 |
| **J13** | 20 | 22 | 14 | 38 |
| **J14** | 16 | 19 | 10 | 34 |
| **J15** | 13 | 15 | 8 | 31 |
| **J16** | 11 | 11 | 4 | 28 |
| **J17** | 8 | 7 | 0 | 25 |
| **J18** | 5 | 4 | | 23 |
| **J19** | 1 | 2 | | 20 |
| **J20** | 1 | 0 | | 16 |
| **J21** | 0 | | | 13 |
| **J22** | | | | 11 |
| **J23** | | | | 7 |
| **J24** | | | | 5 |
| **J25** | | | | 4 |
| **J26** | | | | 1 |
| **J27** | | | | 1 |
| **J28** | | | | 0 |

Dans cette étude, les oeufs pondus ont été soumis à une surveillance particulière et on a trouvé que le nombre d'oeufs pondus dans le groupe contrôle est plus élevé par rapport aux groupes administrés avec de la doxycycline (tableau 2 ci-après). En outre, on observe une diminution de la ponte des oeufs dans chaque groupe de poux (figure 1). Ces données pourraient s'expliquer par le fait que le nombre d'oeufs pondus par pou et par jour est corrélé à l'état nutritionnel (quantité de sang plus la concentration d'antibiotique ingérée) du pou. Mais par contre, la production moyenne de lentes n'est pas significativement différente entre les 4 groupes (ANOVA test, p-value = 0,97).

De ces résultats, on peut conclure que la doxycycline peut réduire la durée de vie et la fertilité des poux.

| **Tableau 2. surveillance des lentes récupérées par jour** | | | | |
|---|---|---|---|---|
| | **Doxy 10 µg/ml** | **Doxy 20 µg/ml** | **Doxy 50 µg/ml** | **C** |
| **J0** | 0 | 0 | 0 | 0 |
| **J1** | 59 | 62 | 58 | 72 |
| **J2** | 46 | 60 | 51 | 65 |
| **J3** | 42 | 67 | 54 | 66 |
| **J4** | 56 | 49 | 52 | 60 |
| **J5** | 45 | 55 | 45 | 52 |
| **J6** | 36 | 49 | 44 | 40 |
| **J7** | 38 | 53 | 48 | 51 |
| **J8** | 44 | 49 | 42 | 45 |
| **J9** | 50 | 52 | 43 | 58 |
| **J10** | 45 | 41 | 31 | 51 |
| **J11** | 44 | 34 | 27 | 45 |
| **J12** | 35 | 37 | 33 | 36 |
| **J13** | 33 | 33 | 24 | 38 |
| **J14** | 29 | 31 | 25 | 34 |
| **J15** | 23 | 20 | 15 | 28 |
| **J16** | 25 | 15 | 10 | 27 |
| **J17** | 21 | 16 | 4 | 30 |
| **J18** | 16 | 14 | 0 | 24 |
| **J19** | 9 | 10 | 0 | 20 |
| **J20** | 3 | 4 | | 15 |
| **J21** | 0 | 0 | | 14 |
| **J22** | | | | 10 |
| **J23** | | | | 4 |
| **J24** | | | | 2 |
| **J25** | | | | 0 |
| **J26** | | | | 0 |

On a développé dans cette étude une nouvelle analyse de déconvolution spectrale à l'aide du logiciel MetaMorph (version 4.6, Molecular Devices, USA) comme décrit ci-dessus. On observe que la fluorescence des lots témoins négatifs est supérieure par rapport aux lots traités avec la doxycycline (tableau 3 ci-après). Ceci s'explique par le passage direct de la doxycycline à travers le mycétome des poux tel que montré par analyse par FISH.

La diminution de la florescence bactérienne symbiotique pourrait confirmer la mort de la bactérie hôte. Cette approche a permis de visualiser des signaux de fluorescence spécifiques, une fluorescence spécifique de la bactérie distincte du signal d'autofluorsecence du pou due à la présence de chitine. On peut conclure que l'ingestion de la doxycycline à différentes doses affecte l'endosymbionte du pou à travers le mycétome des poux tel que démontré par l'analyse par FISH.

Il existe une corrélation entre la consommation de doxycycline, et la durée de vie de poux. Pour montrer la corrélation entre la consommation de doxycycline et la durée de vie des poux, on a suivi les quatre lots jusqu'à épuisement, puis on a comparé le ratio moyen des fluorescences de dix poux pris au hasard dans chacun des différents groupes en parallèle avec dix poux vivants comme contrôles (contrôles vivants) (tableau 3). On constate que les quatre tendances d'évolution sont significativement différentes (Test Logrank: p-valeur = 3.7x10-10). Les délais moyens de survie dans le groupe contrôle et la doxycycline 10, 20, 50 µg/ml sont respectivement = 13.3 jours ; 8.7 jours ; 8.6 jours et 7.2 jours. La survie moyenne diffère selon le groupe (p-value = 9.5e-07, test de Kruskal-Wallis). De plus, les résultats montrent que la fluorescence moyenne (MF) dans le groupe doxycycline 10, 20 et 50 µg/ml était de MF = 10.1; 5.3 et 1.4 de 512 pixels respectivement. Dans le groupe contrôle vivant et mort, cette fluorescence moyenne était de 13.9 et 12.4 de 512 pixels respectivement. L'écart-type (SD) dans le groupe doxycycline 10, 20 et 50 µg/ml est SD = [1.8], [1.5] et [0.6] respectivement. Dans le groupe contrôle vivant et mort, l'écart type est SD = [2.2] et [1.9] respectivement. Les intervalles de confiance (IC) dans les groupes doxycycline 10, 20 et 50 µg/ml sont IC = [8.9; 11.4], [4.2; 6.4] et [0.9; 1.9] respectivement. Dans le groupe contrôle vivant et mort, les intervalles de confiance sont IC = [12.3; 15.5] et [11.0; 13.8], respectivement. On constate que plus la dose de doxycycline est élevée, moins la fluorescence est fiable, et la mortalité est élevée (figure 2). A la lumière de ces résultats, on peut conclure que l'ingestion de la doxycycline à différentes doses affecte la durée de vie des poux.

| **Tableau 3. Mesure de l'intensité de la florescence dans les 4 populations de poux** | | | | |
|---|---|---|---|---|
| **Doxycycline** | **Jours** | **Surface totale** | **Surface marquée** | **Ratio d'intensité** |
| **Dose 10µg/ml** | J1 | 24465 | 2799 | **11,4** |
| | J2 | 30185 | 3316 | **10,9** |
| | J3 | 12391 | 1545 | **12,4** |
| | J4 | 14769 | 1350 | **9,1** |
| | J5 | 25025 | 2961 | **11,8** |
| | J6 | 13837 | 1199 | **8,6** |
| | J7 | 14128 | 1650 | **11,6** |
| | J8 | 33566 | 2523 | **7,5** |
| | J9 | 25128 | 2650 | **10,5** |
| | J10 | 22399 | 1706 | **7,6** |
| | | | | |
| **Dose 20µg/ml** | J1 | 4808 | 325 | **6,7** |
| | J2 | 16217 | 1205 | **7,4** |
| | J3 | 29211 | 1537 | **5,2** |
| | J4 | 19730 | 1190 | **6** |
| | J5 | 37185 | 1231 | **3,3** |
| | J6 | 32994 | 2179 | **6,6** |
| | J7 | 51047 | 2468 | **4,8** |
| | J8 | 16231 | 395 | **2,4** |
| | J9 | 29970 | 1385 | **5,9** |
| | J10 | 20479 | 1216 | **4,6** |
| | | | | |
| **Dose 50µg/ml** | J1 | 54465 | 1033 | **1,8** |
| | J2 | 39619 | 971 | **2,4** |
| | J3 | 43992 | 766 | **1,7** |
| | J4 | 29838 | 656 | **2,1** |
| | J5 | 34239 | 520 | **1,5** |
| | J6 | 47080 | 329 | **0,7** |
| | J7 | 30240 | 488 | **1,6** |
| | J8 | 52023 | 479 | **0,9** |
| | J9 | 49892 | 284 | **0,5** |
| | J10 | 36053 | 428 | **1,1** |
| | | | | |
| **Contrôles vivants** | 1 | 7558 | 1249 | **16,5** |
| | 2 | 13091 | 1898 | **14,4** |
| | 3 | 11058 | 1467 | **13,2** |
| | 4 | 12861 | 1943 | **15,1** |
| | 5 | 10913 | 1134 | **10,3** |
| | 6 | 8962 | 1580 | **17,6** |
| | 7 | 12496 | 1605 | **12,8** |
| | 8 | 12381 | 1397 | **11,2** |
| | 9 | 9914 | 1290 | **13** |
| | 10 | 7945 | 1156 | **14,5** |
| | | | | |
| **Contrôles morts** | 1 | 9488 | 1358 | **14,3** |
| | 2 | 9523 | 1275 | **13,3** |
| | 3 | 12675 | 1269 | **10** |
| | 4 | 8977 | 1462 | **16,2** |
| | 5 | 9173 | 1051 | **11,4** |
| | 6 | 9923 | 986 | **9,9** |
| | 7 | 9873 | 1280 | **12,9** |
| | 8 | 10506 | 1163 | **11** |
| | 9 | 13279 | 1755 | **13,2** |
| | 10 | 12391 | 1518 | **12,2** |

### Exemple 2: Essais comparatifs avec du sang contenant de l'ivermectine et/ou un antibiotique établissant la non résistance et l'effet synergique ivermectine/antibiotique sur la survie des poux et la production des lentes.

### 1) Matériels et méthodes.

Les matériels et méthodes étaient les mêmes que pour l'exemple 1.

Pour la préparation de repas de sang avec l'ivermectine et les antibiotiques, du sang humain Groupe A rhésus positif (A+) de l'Établissement Français du Sang (EFS) a été utilisé. Le Sang a été distribué dans les tubes de 5 ml et conservé à -20° C avant de l'utiliser. D'abord il est chauffé dans un bain humide à 37° C pendant 30 minutes. Puis, dans un volume de 1,485 ml de sang hémolysé pour chacun des trois groupes de poux, il a été ajouté 15 µl d'ivermectine et d'antibiotiques (solutions injectables utilisées) à des concentrations différentes de 50 ng/ml pour l'ivermectine, et 20, 10, 8 et 4 µg/ml pour la doxycycline, la rifampicine, l'azithromycine et l'érythromycine respectivement.

Pour le suivi des poux, 585 poux adultes (comprenant des mâles et des femelles) répartis en 13 groupes de 45 poux ont été mis sur un morceau de tissu noir conservé dans des pots et maintenu à l'étuve à 29° C et 90% d'humidité.

Dans une première série d'expérience, 3 groupes de poux ont été nourris avec du sang contenant seulement l'un des composés ivermectine, doxycycline, rifampicine et érythromycine et Un cinquième groupe de poux représente un témoin négatif nourri exclusivement avec du sang sans antibiotique.

Dans une deuxième série d'expérience, 4 groupes de poux ont été nourris avec du sang contenant respectivement l'ivermectine seule, et la combinaison de l'ivermectine et un antibiotique à des concentrations différentes (50 ng/ml pour l'ivermectine et 20,10 et 4 µg/ml pour la doxycycline, la rifampicine et l'érythromycine respectivement). Un cinquième groupe de poux représente un témoin négatif nourri exclusivement avec du sang.

Dans une troisième série d'expérience, trois groupes de poux ont été nourris avec du sang contenant : (a) l'azithromycine seule, (b) l'ivermectine seule, (c) la combinaison de l'ivermectine et l'azithromycine, les concentrations étant : 50 ng/ml pour l'ivermectine et 8 µg/ml pour l'azithromycine). Un quatrième groupe de poux représente un témoin négatif nourri exclusivement avec du sang sans antibiotique.

Les poux ont été mis tous les jours sur la membrane pour une heure de repas de sang. Chaque jour, la mortalité a été notée dans chacun des groupes. Cette expérience a été reproduite trois fois.

### 2) Résultats.

Chaque groupe de poux a été suivi jusqu'à terme et le taux d'alimentation sanguin était supérieur à 90%.

Dans la première série d'expériences, pour les groupes traités avec la doxycycline, la rifampicine, l'érythromycine seules la survie était d'au moins 12 jours pour la doxycycline et la rifampicine, 13 jours pour l'érythromycine, le groupe contrôle ayant survécu pendant 17 jours.

Dans la deuxième série d'expériences, le groupe traité avec l'association ivermectine/doxycycline a survécu seulement 5 jours, le groupe traité avec ivermectine/rifampicine a survécu 6 jours, le groupe traité avec ivermectine/erythromycine a survécu 7 jours et le groupe traité avec ivermectine seule a survécu pendant 8 jours, Enfin le groupe contrôle a survécu pendant 17 jours.

Dans la troisième série d'expériences, le groupe traité avec ivermectine/azithromycine a survécu 8 jours et le groupe traité avec ivermectine seule a survécu pendant 9 jours, et le groupe traité par la seule azithromycine a survécu pendant 15 jours et le groupe contrôle a survécu pendant 18 jours.

Pour la doxycycline, la rifampicine, l'érythromycine, les nombres de poux récupérés chaque jour suite à un traitement avec un antibiotique seul sont rapportés dans le tableau 1A et avec une combinaison ivermectine + antibiotique dans le tableau 2A.

Pour l'azithromycine, les nombres de poux récupérés chaque jour suite à un traitement avec un antibiotique seul et avec une combinaison ivermectine + antibiotique sont rapportés dans le tableau 3A.

Au total, le délai maximum de survie parmi les groupes traités par une association ivermectine/antibiotique a été de 7 ou 8 jours.

A la lumière de ces résultats, on peut conclure que l'effet de l'association ivermectine/antibiotiques est létale plus rapidement pour les poux que pour les antibiotiques pris seuls et l'ivermectine seule.

Par ailleurs les lentes pondues ont été aussi l'objet d'un comptage particulier. Ainsi, on observe dans chacun des groupes traités, une diminution très progressive dans la production de lentes comparativement au groupe contrôle (tableaux 1B à 3B ci-après).

Le nombre de lentes récupéré chaque jour suite à un traitement avec un antibiotique seul est rapporté dans le tableau 1B et avec une combinaison d'ivermectine et d'antibiotique dans le tableau 2B et 3B.

Dans les tableaux, C= contrôle ou témoin négatif, Iverm = ivermectine à 50 ng/ml et Rifam = rifampicine à 10 µg/ml, Dox=doxycycline à 20 µg/ml, Erythro = erythromycine à 4 µg/ml, Azithro = azithromycine à 8 µg/ml.

La diminution de la reproduction établissant la supériorité de l'association par rapport à la doxycycline seule et l'ivermectine seule pourrait s'expliquer par la potentialisation de l'effet inhibiteur de l'association sur les organes reproducteurs du pou, entrainant la diminution de la ponte des oeufs et démontrant ainsi la supériorité de l'association.

| **Tableau 1A.** Nombre de poux récupérés | | | | |
|---|---|---|---|---|
| **J** | **Doxy** | **Erythro** | **Rifam** | **Contrôle** |
| **j0** | 45 | 45 | 45 | 45 |
| **j1** | 40 | 41 | 40 | 44 |
| **j2** | 36 | 38 | 38 | 40 |
| **j3** | 33 | 33 | 34 | 38 |
| **j4** | 29 | 30 | 30 | 37 |
| **j5** | 24 | 27 | 26 | 36 |
| **j6** | 21 | 24 | 23 | 34 |
| **j7** | 17 | 20 | 21 | 32 |
| **j8** | 15 | 18 | 18 | 29 |
| **j9** | 12 | 15 | 14 | 25 |
| **j10** | 9 | 12 | 10 | 22 |
| **j11** | 6 | 8 | 5 | 21 |
| **j12** | 2 | 7 | 1 | 18 |
| **j13** | 0 | 3 | 0 | 14 |
| **j14** | | 0 | | 13 |
| **j15** | | | | 9 |
| **j16** | | | | 5 |
| **j17** | | | | 1 |
| **j18** | | | | 0 |

| **Tableau 1B**. Nombre de lentes récupérées | | | | |
|---|---|---|---|---|
| **J** | **Doxy** | **Erythro** | **Rifam** | **Contrôle** |
| **j0** | 0 | 0 | 0 | 0 |
| **j1** | 35 | 37 | 33 | 32 |
| **j2** | 24 | 23 | 22 | 27 |
| **j3** | 15 | 30 | 18 | 23 |
| **j4** | 22 | 17 | 20 | 26 |
| **j5** | 18 | 11 | 16 | 22 |
| **j6** | 13 | 8 | 13 | 25 |
| **j7** | 9 | 12 | 10 | 17 |
| **j8** | 11 | 9 | 12 | 20 |
| **j9** | 7 | 11 | 9 | 17 |
| **j10** | 2 | 8 | 3 | 13 |
| **j11** | 0 | 4 | 0 | 15 |
| **j12** | | 1 | | 10 |
| **j13** | | 0 | | 11 |
| **j14** | | | | 7 |
| **J15** | | | | 3 |
| **j16** | | | | 0 |

| **Tableau 2A.** Nombre de poux récupérés | | | | | |
|---|---|---|---|---|---|
| **J** | **Iverm** | **Doxy** + **Iverm** | **Erytho + Iverm** | **Rifam + Iverm** | **Contrôle** |
| **j0** | 45 | 45 | 45 | 45 | 45 |
| **j1** | 37 | 34 | 38 | 39 | 44 |
| **j2** | 29 | 25 | 33 | 31 | 40 |
| **j3** | 21 | 16 | 25 | 21 | 38 |
| **j4** | 17 | 9 | 18 | 15 | 37 |
| **j5** | 11 | 3 | 11 | 10 | 36 |
| **j6** | 8 | 0 | 6 | 3 | 34 |
| **j7** | 3 | | 2 | 0 | 32 |
| **j8** | 1 | | 0 | | 29 |
| **j9** | 0 | | | | 25 |
| **j10** | | | | | 22 |
| **j11** | | | | | 21 |
| **j12** | | | | | 18 |
| **j13** | | | | | 14 |
| **j14** | | | | | 13 |
| **j15** | | | | | 9 |
| **j16** | | | | | 5 |
| **j17** | | | | | 1 |
| **j18** | | | | | 0 |

| **Tableau 2B.** Nombre de lentes récupérées | | | | | |
|---|---|---|---|---|---|
| **J** | **Iverm** | **Doxy + Iverm** | **Erytho + Iverm** | **Rifam + Iverm** | **Contrôle** |
| **j0** | 0 | 0 | 0 | 0 | 0 |
| **j1** | 19 | 31 | 29 | 41 | 32 |
| **j2** | 13 | 16 | 18 | 23 | 27 |
| **j3** | 10 | 10 | 8 | 13 | 23 |
| **j4** | 9 | 2 | 11 | 5 | 26 |
| **j5** | 2 | 2 | 6 | 1 | 22 |
| **j6** | 0 | 0 | 3 | 0 | 25 |
| **j7** | | | 0 | | 17 |
| **j8** | | | | | 20 |
| **j9** | | | | | 17 |
| **j10** | | | | | 13 |
| **j11** | | | | | 15 |
| **j12** | | | | | 10 |
| **j13** | | | | | 11 |
| **j14** | | | | | 7 |
| **j15** | | | | | 3 |
| **j16** | | | | | 0 |

| **Tableau 3A: Nombre de poux récupérés** | | | | |
|---|---|---|---|---|
| jours | Azithro | Iverm | Azithro + Iverm | C |
| J0 | 45 | 45 | 45 | 45 |
| J1 | 44 | 42 | 41 | 43 |
| J2 | 39 | 37 | 35 | 42 |
| J3 | 37 | 31 | 29 | 41 |
| J4 | 36 | 25 | 27 | 39 |
| J5 | 31 | 20 | 18 | 37 |
| J6 | 28 | 15 | 12 | 34 |
| J7 | 28 | 10 | 8 | 32 |
| J8 | 25 | 6 | 2 | 29 |
| J9 | 22 | 2 | 0 | 26 |
| J10 | 20 | 0 | | 23 |
| J11 | 17 | | | 21 |
| J12 | 15 | | | 18 |
| J13 | 10 | | | 16 |
| J14 | 7 | | | 13 |
| J15 | 3 | | | 10 |
| J16 | 0 | | | 7 |
| J17 | | | | 7 |
| J18 | | | | 3 |
| J19 | | | | 0 |

| **Tableau 3B: Nombre de lentes récupérées** | | | | |
|---|---|---|---|---|
| jours | Azithro | Iverm | Azithro + Iverm | C |
| J0 | 0 | 0 | 0 | |
| J1 | 37 | 28 | 30 | 35 |
| J2 | 30 | 33 | 29 | 32 |
| J3 | 21 | 23 | 18 | 28 |
| J4 | 17 | 15 | 11 | 19 |
| J5 | 12 | 10 | 7 | 12 |
| J6 | 20 | 5 | 2 | 14 |
| J7 | 13 | 3 | 0 | 15 |
| J8 | 16 | | | 18 |
| J9 | 12 | | | 19 |
| J10 | 9 | | | 15 |
| J11 | 11 | | | 13 |
| J12 | 13 | | | 6 |
| J13 | 3 | | | 10 |
| J14 | 6 | | | 12 |
| J15 | 3 | | | 7 |
| J16 | 0 | | | 2 |
| J17 | | | | 0 |
| J18 | | | | |
| J19 | | | | |

### Exemple 3 : traitement de patients atteints de pédiculoses.

Ces résultats ont conduit à spécifier les séquences suivantes de traitement de 7 jours pour des patients atteints de pédiculose capillaire ou corporelle avec les doses unitaires journalières suivantes administrées de façon orale (les doses journalières pour la rifampicine et l'ivermectine étant rapportées au poids du patient):
1) Séquence n°1 pour un adulte
   - 1° jour : une dose d'ivermectine de 100 à 200 µg/kg,
   - 2° au 6° jour : une dose de 125 à 200 mg de doxycycline, et/ou une dose de 5 à 20 mg/kg de rifampicine, et/ou une dose de 10 à 20 g/kg d'érythromycine, et/ou une dose de 250 à 500 mg d'azithromycine.
   - 7° jour : une dose d'ivermectine de 100 à 200 µg/kg.
2) Séquence n°2 pour un adulte
   - 1° jour : une dose d'ivermectine de 100 à 200 µg/kg et une dose de 125 à 200 mg de doxycycline, et/ou une dose de 5 à 20 mg/kg de rifampicine, et/ou une dose de 1 à 2 g d'érythromycine, et/ou une dose de 250 à 500 mg d'azithromycine, et
   - 2° au 6° jour : une dose de 125 à 200 mg de doxycycline de et/ou une dose de 5 à 20 mg/kg de rifampicine et/ou une dose de 1 à 2 g d'érythromycine et/ou une dose de 250 à 500 mg d'azithromycine, et
   - 7° jour : une dose d'ivermectine de 100 à 200 µg/kg et une dose de 125 à 200 mg/j de doxycycline et/ou une dose de 5 à 20 mg/kg de rifampicine, et/ou une dose de 1 à 2 mg/kg d'érythromycine et/ou une dose de 250 à 500 mg d'azithromycine.
3) Séquence pour un enfant (moins de 15 ans)
   On mettra en oeuvre les mêmes séquences n° 1 ou 2 ci-dessus mais sans doxycycline et avec les doses suivantes de :
   - 5 à 20 mg/kg de rifampicine, et/ou
   - 20 à 30 mg/kg/jour d'érythromycine rapporté au poids de l'enfant, et/ou
   - 10 à 20 mg/kg/jour d'azithromycine rapporté au poids de l'enfant sans dépasser la posologie de 500 mg/jour.

### BIBLIOGRAPHIE

1. Chosidow O, Giraudeau B. Topical ivermectin lotion for head lice. N Engl J Med. 2013 Mar 7;368 (10):968.
2. Badiaga S, Foucault C, Rogier C, Doudier B, Rovery C, Dupont HT, Castro P, Raoult D, Brouqui P. The effect of a single dose of oral ivermectin on pruritus in the homeless. J Antimicrob Chemother. 2008 Aug; 62(2):404-9.
3. Burgess IF, Barker SC, Mumcuoglu KY. Topical ivermectin lotion for head lice. N Engl J Med. 2013 Mar 7;368 (10):966-7.
4. Kirkness et al Genome sequences of the human body louse and its primary endosymbiont provide insights into the permanent parasitic lifestyle. Proc Natl Acad Sci USA. 2010 Jul 6;107(27):12168-73.
5. Culpepper GH (1944) The rearing and maintenance of a laboratory colony of the body louse. Am J Trop Med Hyg 24:327-329.
6. Culpepper, G. H (1946) Rearing body lice on rabbits: J.Econ.Entomol., v. 39, no. 5, p. 660.
7. Culpepper, G. H (1948) Rearing and maintaining a laboratory colony of body lice on rabbits: Am.J.Trop.Med.Hyg., v. 28, no. 3, p. 499-504.
8. O'Neill S. L., Hoffmann A. A. & Werren J. H (1997) Influential Passengers: inherited microorganisms and arthropod reproduction. Oxford University Press., New York, USA, 214 p.
9. Coulibaly YI, et al. (2009) A randomized trial of doxycycline for Mansonella perstans infection N.Engl.J.Med, v. 361, no. 15, p. 1448-1458.
10. Girin C and Bouletreau M (1995) Microorganisms-associated variation in host infestation efficiency in a parasitoid wasp Trichogramma bourarachae. Experientia 52:398-402.
11. Perotti, M. A., J. M. Allen, D. L. Reed, and H. R. Braig (2007) Host-symbiont interactions of the primary endosymbiont of human head and body lice: FASEB J, v. 21, no. 4, p.1058-1066.

## Revendications

1. Association d'au moins un composé antibiotique choisi parmi la doxycycline, la rifampicine, l'érythromycine et l'azithromycine et d'un composé antiparasitaire consistant dans l'ivermectine pour une utilisation dans un traitement thérapeutique contre les poux.

2. Association pour une utilisation selon une revendication 1 pour traiter une pédiculose corporelle ou capillaire liée à la présence de poux.

3. Association pour une utilisation selon une des revendications 1 ou 2 pour une action létale contre les poux et/ou diminuer leur reproduction.

4. Association pour une utilisation selon l'une des revendications 1 à 3 pour une utilisation simultanée, séparée ou étalée dans le temps du (ou des)dit(s) composé(s) antibiotique(s) et dudit composé antiparasitaire.

5. Association pour une utilisation selon l'une des revendications 1 à 4 dans laquelle ledit composé antibiotique est choisi parmi la doxycycline et la rifampicine.

6. Association pour une utilisation selon l'une des revendications 1 à 4 pour un traitement d'un enfant de moins de 15 ans dans laquelle ledit composé antibiotique est autre que la doxycycline.

7. Association pour une utilisation selon la revendication 6 pour le traitement d'un enfant de moins de 15 ans dans laquelle ledit composé antibiotique est choisi parmi l'érythromycine et l'azithromycine.

8. Composition pharmaceutique comprenant une association d'au moins un dit composé antibiotique et un dit composé antiparasitaire en quantités thérapeutiquement efficaces pour une utilisation dans un traitement contre les poux selon l'une des revendications 1 à 7.

9. Composition pharmaceutique comprenant une association pour une utilisation dans un traitement contre les poux selon la revendication 8 **caractérisée en ce qu'**elle se présente sous forme administrable par voie orale ou par application topique cutanée ou capillaire.

10. Ensemble de compositions pharmaceutiques pour une utilisation dans un traitement contre les poux selon l'une des revendications 8 ou 9 **caractérisé en ce qu'**il comprend une pluralité de doses journalières ou doses unitaires de compositions pharmaceutiques de dits composé(s) antibiotique(s) et dit composé antiparasitaire en quantités utiles pour être appliquées dans un traitement de plusieurs jours, comprenant :
- une pluralité de doses unitaires ou journalières de compositions pharmaceutiques comprenant au moins dit composé antiparasitaire, et
- une pluralité de doses unitaires ou journalières de compositions pharmaceutiques comprenant au moins un dit composé antibiotique.

11. Ensemble de compositions pharmaceutiques pour une utilisation dans le traitement contre les poux selon la revendication 10 **caractérisé en ce qu'**il comprend une pluralité de doses unitaires de dit(s) composé(s) antibiotique(s) et dit composé antiparasitaire en quantités utiles pour être appliquées dans un traitement de plusieurs jours, comprenant :
- une pluralité de doses unitaires ou journalières de compositions pharmaceutiques d'un mélange de dits composé(s) antibiotique(s) et de dit composé antiparasitaire, et
- une pluralité de doses unitaires ou journalières de compositions pharmaceutiques de composé(s) antibiotique(s) comme seul agent actif létal contre les poux.

12. Ensemble de compositions pharmaceutiques pour une utilisation contre les poux selon l'une des revendications 10 à 11 comprenant au moins un dit composé antibiotique et dit composé antiparasitaire en quantités utiles pour être appliquées dans un traitement de 7 jours, comprenant :
- deux doses unitaires journalières de mélange d'ivermectine et d'au moins un composé antibiotique choisi parmi la doxycycline, l'azithromycine, l'érythromycine et la rifampicine pour les 1er et 7ème jours,
- 5 doses unitaires journalières d'au moins un composé antibiotique choisi parmi la doxycycline, l'érythromycine, l'azithromycine et la rifampicine, sans ivermectine, pour respectivement les 5 jours suivants le premier jour.

13. Ensemble de compositions pharmaceutiques pour une utilisation dans un traitement contre les poux selon la revendication 11 ou 12 pour un traitement de 7 jours, comprenant :
- une combinaison d'ivermectine et un seul dit composé antibiotique le 1er jour,
- un seul dit composé antibiotique sans ivermectine pendant les 5 jours suivants, et
- un 2ème traitement avec une combinaison d'ivermectine et un seul dit composé antibiotique le 7ème jour.

14. Ensemble de compositions pharmaceutiques pour une utilisation dans un traitement contre les poux selon la revendication 13 **caractérisé en ce que** le dit composé antibiotique est choisi parmi l'érythromycine et l'azithromycine.

15. Ensemble de compositions pharmaceutiques pour une utilisation dans un traitement contre les poux selon la revendication 13 **caractérisé en ce que** le dit composé antibiotique est choisi parmi la doxycycline et la rifampicine.

## Patentansprüche

1. Kombination aus mindestens einer antibiotischen Verbindung, ausgewählt aus Doxycyclin, Rifampicin, Erythromycin und Azithromycin, und einer antiparasitären Verbindung, bestehend aus Ivermectin für eine Verwendung bei einer therapeutischen Behandlung gegen Läuse.

2. Kombination für eine Verwendung gemäß Anspruch 1 zur Behandlung einer Pedikulose von Körper oder Haaren im Zusammenhang mit Läusen.

3. Kombination für eine Verwendung gemäß Anspruch 1 oder 2 zur tödlichen Bekämpfung von Läusen und/oder zur Verminderung ihrer Vermehrung.

4. Kombination für eine Verwendung gemäß einem der Ansprüche 1 bis 3 zur gleichzeitigen, getrennten oder zeitlich gestaffelten Verwendung der antibiotischen Verbindung(en) und der antiparasitären Verbindung.

5. Kombination für eine Verwendung gemäß einem der Ansprüche 1 bis 4, wobei die antibiotische Verbindung aus Doxycyclin und Rifampicin ausgewählt ist.

6. Kombination für eine Verwendung gemäß einem der Ansprüche 1 bis 4 für eine Behandlung eines Kindes unter 15 Jahren, wobei die antibiotische Verbindung nicht Doxycyclin ist.

7. Kombination für eine Verwendung gemäß Anspruch 6 für die Behandlung eines Kindes unter 15 Jahren, wobei die antibiotische Verbindung aus Erythromycin und Azithromycin ausgewählt ist.

8. Pharmazeutische Zusammensetzung, umfassend eine Kombination aus mindestens einer der antibiotischen Verbindungen und einer der antiparasitären Verbindungen in therapeutisch wirksamen Mengen für eine Verwendung bei einer Behandlung gegen Läuse gemäß einem der Ansprüche 1 bis 7.

9. Pharmazeutische Zusammensetzung, umfassend eine Kombination für eine Verwendung bei einer Behandlung gegen Läuse gemäß Anspruch 8, **dadurch gekennzeichnet, dass** sie in einer Form vorliegt, die entweder oral oder durch topische Anwendung auf Haut oder Haar verabreichbar ist.

10. Satz von pharmazeutischen Zusammensetzungen für eine Verwendung bei einer Behandlung gegen Läuse gemäß einem der Ansprüche 8 oder 9, **dadurch gekennzeichnet, dass** er eine Vielzahl von Tages- oder Einzeldosen von pharmazeutischen Zusammensetzungen der antibiotischen Verbindung(en) und der antiparasitären Verbindung in Mengen umfasst, die zur Anwendung bei einer mehrtägigen Behandlung geeignet sind, umfassend:
- eine Vielzahl von Einzel- oder Tagesdosen von pharmazeutischen Zusammensetzungen, umfassend mindestens die antiparasitäre Verbindung, und
- eine Vielzahl von Einzel- oder Tagesdosen von pharmazeutischen Zusammensetzungen, umfassend mindestens eine der antibiotischen Verbindungen.

11. Satz von pharmazeutischen Zusammensetzungen für eine Verwendung bei der Behandlung gegen Läuse gemäß Anspruch 10, **dadurch gekennzeichnet, dass** er eine Vielzahl von Einzeldosen der antibiotischen Verbindung(en) und der antiparasitären Verbindung in Mengen umfasst, die zur Anwendung bei einer mehrtägigen Behandlung geeignet sind, umfassend:
- eine Vielzahl von Einzel- oder Tagesdosen von pharmazeutischen Zusammensetzungen aus einer Mischung der antibiotischen Verbindung(en) und der antiparasitären Verbindung und
- eine Vielzahl von Einzel- oder Tagesdosen von pharmazeutischen Zusammensetzungen von (einer) antibiotischen Verbindung(en) als einzigem tödlichen Wirkstoff gegen Läuse.

12. Satz von pharmazeutischen Zusammensetzungen für eine Verwendung gegen Läuse gemäß einem der Ansprüche 10 bis 11, umfassend mindestens eine antibiotische Verbindung und die antiparasitäre Verbindung in Mengen, die zur Anwendung bei einer 7-tägigen Behandlung geeignet sind, umfassend:
- zwei einzelne Tagesdosen einer Mischung von Ivermectin und mindestens einer antibiotische Verbindung, ausgewählt aus Doxycyclin, Azithromycin, Erythromycin und Rifampicin für den ersten und siebten Tag,
- 5 einzelne Tagesdosen mindestens einer antibiotischen Verbindung, ausgewählt aus Doxycyclin, Erythromycin, Azithromycin und Rifampicin, ohne Ivermectin, jeweils für die 5 Tage nach dem ersten Tag.

13. Satz von pharmazeutischen Zusammensetzungen für eine Verwendung bei einer Behandlung gegen Läuse gemäß Anspruch 11 oder 12 für eine 7-tägige Behandlung, umfassend:
- eine Kombination aus Ivermectin und einer einzigen der antibiotischen Verbindungen am ersten Tag,
- eine einzige der antibiotischen Verbindungen ohne Ivermectin für die nächsten 5 Tage und
- eine 2. Behandlung mit einer Kombination aus Ivermectin und der einzigen der antibiotischen Verbindungen am 7 Tag.

14. Satz von pharmazeutischen Zusammensetzungen für eine Verwendung bei einer Behandlung gegen Läuse gemäß Anspruch 13, **dadurch gekennzeichnet, dass** die antibiotische Verbindung aus Erythromycin und Azithromycin ausgewählt ist.

15. Satz von pharmazeutischen Zusammensetzungen für eine Verwendung bei einer Behandlung gegen Läuse gemäß Anspruch 13, **dadurch gekennzeichnet, dass** die antibiotische Verbindung aus Doxycyclin und Rifampicin ausgewählt ist.

## Claims

1. Association of at least one antibiotic compound selected from among doxycycline, rifampicin, erythromycin and azithromycin, and of an antiparasitic compound that is ivermectin, for use in therapeutic treatment against lice.

2. Association for use as in claim 1 to treat body or capillary pediculosis related to the presence of lice.

3. Association for use as in one of claims 1 or 2 for lethal action against lice and/or to reduce the reproduction thereof.

4. Association for use as in one of claims 1 to 3 for simultaneous, separate or time-staggered use of said antibiotic compound(s) and of said antiparasitic compound.

5. Association for use as in one of claims 1 to 4, wherein said antibiotic compound is selected from among doxycycline and rifampicin.

6. Association for use as in one of claims 1 to 4 to treat children under the age of 15 years, wherein said antibiotic compound is other than doxycycline.

7. Association for use as in claim 6 to treat children under the age of 15 years, wherein said antibiotic compound is selected from among erythromycin and azithromycin.

8. Pharmaceutical composition comprising an association of at least one said antibiotic compound and said antiparasitic compound in therapeutically efficient amounts for use in treatment against lice according to one of claims 1 to 7.

9. Pharmaceutical composition comprising an association for use in treatment against lice according to claim 8, **characterized in that** it is in a form that can be administered via oral route or via topical cutaneous or capillary application.

10. Set of pharmaceutical compositions for use in treatment against lice according to one of claims 8 or 9, **characterized in that** it comprises a plurality of daily doses or unit doses of pharmaceutical compositions of said antibiotic compound(s) and said antiparasitic compound in amounts useful for application in treatment over several days, comprising:
- a plurality of unit or daily doses of pharmaceutical compositions comprising at least said antiparasitic compound; and
- a plurality of unit or daily doses of pharmaceutical compositions comprising at least one said antibiotic compound.

11. Set of pharmaceutical compositions for use in treatment against lice according to claim 10, **characterized in that** it comprises a plurality of unit doses of said antibiotic compound(s) and said antiparasitic compound in amounts useful for application in treatment over several says, comprising:
- a plurality of unit or daily doses of pharmaceutical compositions of a mixture of said antibiotic compound(s) and said antiparasitic compound; and
- a plurality of unit or daily doses of pharmaceutical compositions of antibiotic compound(s) as sole lethal active agent against lice.

12. Set of pharmaceutical compositions for use against lice according to one of claims 10 to 11 comprising at least one said antibiotic compound and said antiparasitic compound in amounts useful for application in a 7-day treatment, comprising:
- two daily unit doses of a mixture of ivermectin and at least one antibiotic compound selected from among doxycycline, azithromycin, erythromycin and rifampicin for Day 1 and Day 7;
- 5 daily unit doses of at least one antibiotic compound selected from among doxycycline, erythromycin, azithromycin and rifampicin, without ivermectin, for the 5 days respectively following after the first day.

13. Set of pharmaceutical compositions for use in treatment against lice according to claim 11 or 12 for a 7-day treatment, comprising:
- a combination of ivermectin and only one said antibiotic on Day 1;
- only one said antibiotic compound without ivermectin over the 5 following days; and
- a 2^{nd} treatment with a combination of ivermectin and only one said antibiotic compound on Day 7.

14. Set of pharmaceutical compositions for use in treatment against lice according to claim 13, **characterized in that** said antibiotic compound is selected from among erythromycin and azithromycin.

15. Set of pharmaceutical compositions for use in treatment against lice according to claim 13, **characterized in that** said antibiotic compound is selected from among doxycycline and rifampicin.
